# EUROPÄISCHE PATENTSCHRIFT

(11) **EP 1 276 456 B1**
(45) Veröffentlichungstag und Bekanntmachung des Hinweises auf die Patenterteilung: **22.06.2011**
(21) Anmeldenummer: 01971446.8
(22) Anmeldetag: 26.04.2001
(51) Int. Cl.: A61Q 15/00

(54) **DUFTKOMPOSITION AUS HLA-MOLEKÜLEN**
AROMATIC COMPOSITION CONSISTING OF HLA MOLECULES
COMPOSITIONS ODORIFERANTES CONSTITUEES DE MOLECULES D'HLA

(30) Priorität: 27.04.2000 DE 10021579
(43) Veröffentlichungstag der Anmeldung: 22.01.2003
(73) Patentinhaber: Coty B.V., 2031 CC Haarlem (NL)
(72) Erfinder: ZIEGLER, Andreas, 14050 Berlin (DE); UCHANSKA-ZIEGLER, Barbara, 14050 Berlin (DE); GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2001/001609
(87) Internationale Veröffentlichungsnummer: WO 2001/081374

(56) Entgegenhaltungen:
- DATABASE MEDLINE [Online] US NATIONAL LIBRARY OF MEDICINE (NLM), BETHESDA, MD, US; ZIJLSTRA M ET AL: "Germ-line transmission of a disrupted beta 2-microglobulin gene produced by homologous recombination in embryonic stem cells." retrieved from STN Database accession no. 90066674 XP002184707 & NATURE, (1989 NOV 23) 342 (6248) 435-8. ,
- DATABASE MEDLINE [Online] US NATIONAL LIBRARY OF MEDICINE (NLM), BETHESDA, MD, US; BARD J ET AL: "Effect of B2m gene disruption on MHC-determined odortypes." retrieved from STN Database accession no. 2000367630 XP002184708 & IMMUNOGENETICS, (2000 JUN) 51 (7) 514-8. ,
- CHEMICAL ABSTRACTS, vol. 133, no. 6, 7. August 2000 (2000-08-07) Columbus, Ohio, US; abstract no. 71714, EGGERT, F. ET AL: "MHC and olfactory communication in humans" XP002184705 & ADV. CHEM. SIGNALS VERTEBR., [INT. SYMP.], 8TH (1999), MEETING DATE 1997, 181-188. EDITOR(S): JOHNSTON, ROBERT E.;MUELLER-SCHWARZE, DIETLAND; SORENSEN. PETER W. PUBLISHER: KLUWER ACADEMIC/PLENUM PUBLISHERS, NEW YORK N. Y. , 1999,
- CHEMICAL ABSTRACTS, vol. 124, no. 9, 26. Februar 1996 (1996-02-26) Columbus, Ohio, US; abstract no. 114831, SOMMERVILLE, B. A. ET AL: "Volatile identity signals in human axillary sweat: the possible influence of MHC class I genes" XP002184706 & ADV. BIOSCI. (OXFORD) (1995), 93(CHEMICAL SIGNALS IN VERTEBRATES 7), 535- , 1995,

## Beschreibung

Die Erfindung betrifft neue Duftkompositionen, die jeweils zwei unterschiedlichen potentiellen Duftträgern (Partnern) zuzuordnen sind.

Aus dem Stand der Technik sind eine Vielzahl von Düften unterschiedlicher Provenienz bekannt, mit denen von den Anwendern versucht wurde und wird, mittels mehr oder weniger wohlriechender ätherischer öle, Parfüms oder Seifen den vermeintrich ungenügenden eigenen Körpergeruch positiv zu beeinflussen. In den letzten Jahren sind Hinweise bekannt geworden, daß möglicherweise genetisch Ursachen für den einem Individuum eigenen Geruch und damit auch für seine Attraktivität anderen Partnern gegenüber verantwortlich sind. Spezielle Untersuchungen an Probanden (Am. J. Hum. Genet. 61, 505-51 (1997) ließen erkennen, daß die Partnerwahl möglicherweise auf geruchlichem Wege über polymorphe Gene des Haupt-Histokompatibilitätskomplexes (MHC bzw. HLA Komplex beim Menschen) (MHC = major histocompatibility complex; HLA = Human Leucocyte Antigen)) beeinflußt wird. HLA Gene liegen in einem Bereich von etwa 4.000.000 Basenpaaren (bp) auf dem menschlichen Chromosom 6. Es ist bisher noch nicht gelungen, eine eindeutige olfaktorische Zuordnung von Molekülen zu der Entwicklung eines individualspezifischen Geruches zu treffen. Es gibt allerdings Hinweise, daß MHC Moleküle ursächlich an der Erzeugung eines individualspezifischen Geruchsprofils beteiligt sind (Proc. Natl. Acad. Sci. USA 94, 2210-2214, 1997; Proc. Natl. Acad. Sci. USA 96, 1522-1525, 1999.

Der Erfindung liegt die Aufgabe zugrunde, eine Duftkomposition zu entwickeln, die auf Basis einer Auswahl bestimmter Allele von olfaktorisch relevanten Genen und einer nachfolgenden Behandlung ihrer Syntheseprodukte zu geruchsspezifischen Substanzen führt, die in Form von zwei getrennten Formulierungen unterschiedlichen Partnern unterschiedliche aber gegenseitige Attraktivität verleihen sollen.

Erfindungsgemäß wird eine Duftkomposition bereitgestellte.

Duftkomposition aus HLA-Molekulen, hergesteilt nach einem Verfahren, bei dem man
a) HLA Allele der Kiasse I auswählt, die durch in-vitro Mutagenese der B-Tasche bereits vorhandener HLA Allele der Klasse I produziert wurden und natürlicherweise nicht vorkommen und somit eine Haufigkeit von 0 % haben; und
b) den löslichen, extrazellulären Anteil des Proteins, für weiches das ausgewählte Allel kodiert, synthetisiert und dann einer in-vitro Assemblierung in Anwesenheit von (∼2∼ Mikroglobulin (β₂m) unterzieht, indem eine Vielzahl unterschiedlicher Peptide mit einer Lange von typischerweise 7 bis 12 Aminosäuren und mit freien N- und C-Termini zur Ausbildung von löslichen HLA Klasse I Molekülen, bestehend aus den extrazellulären Domänen α₁, α₂ und α₃, β₂m und einem Peptid, entsteht, wobei das Peptid in einer von den extrazellulären Domänen α₁, und α₂ ausgebildeten Peptidbindungstasche des HLA Moleküls gebunden vorliegt; und
c) die gebildeten HLA Klasse I Moleküle mit den gebundenen Peptiden in einer Reinigungsstufe von den anderen Bestandteilen abtrennt; und
d) die gereinigten HLA Klasse I Moleküle einer Fragmentierung mit einer oder mehreren Proteasen unterwirft; und
e) die bei der Fragmentierung entstandenen geruchlich aktiven Stoffe als Einzelkomponente oder als Mischung in eine kosmetische Zubereitung einbringt.

Sowie Verfahren zur Herstellung einer Duftkomposition aus HLA-Molekulen, bei wenigstens
a) unter bekannten HLA Allelen der Klasse I ein Allel auswählt, das sich durch wenigstens ein Merkmal von anderen Allelen der HLA Klasse I Moleküle unterscheidet, das bei weniger als 5 % der Personen der Weltbevölkerung auftritt; oder
b) HLA Allele der Kiasse I auswählt, die durch in-vitro Mutagenese der B-Tasche bereits vorhandener HLA Allele der Klasse I produziert wurden und natürlicherweise nicht vorkommen und somit eine Haufigkeit von 0 % haben; und
c) den löslichen, extrazellulären Anteil des Proteins, für weiches das ausgewählte Allel kodiert, synthetisiert und dann einer in-vitro Assemblierung in Anwesenheit von (∼2∼ Mikroglobulin (β₂m) unterzieht, indem eine Vielzahl unterschiedlicher Peptide mit einer Lange von typischerweise 7 bis 12 Aminosäuren und mit freien N- und C-Termini zur Ausbildung von löslichen HLA Klasse I Molekülen, bestehend aus den extrazellulären Domänen α₁, α₂ und α₃, β₂m und einem Peptid, entsteht, wobei das Peptid in einer von den extrazellulären Domänen α₁, und α₂ ausgebildeten Peptidbindungstasche des HLA Moleküls gebunden vorliegt; und
d) die gebildeten HLA Klasse I Moleküle mit den gebundenen Peptiden in einer Reinigungsstufe von den anderen Bestandteilen abtrennt; und
e) die gereinigten HLA Klasse I Moleküle einer Fragmentierung mit einer oder mehreren Proteasen unterwirft; und
f) die bei der Fragmentierung entstandenen geruchlich aktiven Stoffe als Einzelkomponente oder als Mischung in eine kosmetische Zubereitung einbringt.

Zum besseren Verständnis der Erfindung werden die nachfolgenden Erläuterungen zur intrakorporalen Bildung der HLA Klasse I Moleküle gegeben.

Es ist bekannt, daß Säugetiere die Fähigkeit besitzen, zwischen ihrem eigenen Gewebe und dem anderer Spezies sowie auch dem anderer Individuen ihrer eigenen Spezies zu unterscheiden. Mit Hilfe von Transplantationsstudien bei der Maus konnten auf dem Chromosom 17 in der H-2 Region unterschiedliche Gene identifiziert werden, die für eine rasche Abstoßung des fremden Gewebes ursächlich verantwortlich sind. Dieser Genkomplex ist seitdem als Haupt-Histokompatibilitätskomplex (MHC) bekannt. Der menschliche MHC befindet sich auf dem kurzen Arm des Chromosoms 6 und wird als HLA (Human Leukocyte Antigen) Komplex bezeichnet. Er enthält unter anderem die Gene für die MHC Klasse I Proteine HLA-A, HLA-B und HLA-C sowie die HLA Klasse II Proteine. HLA Moleküle binden intrazellulär Peptide, die beim Abbau cytosolischer (typischerweise MHC Klasse I) oder extrazellulärer Proteine (typischerweise MHC Klasse II) entstehen, und transportieren sie an die Zelloberfläche. Dort können HLA/Peptid Komplexe von den T-Lymphozyten des Immunsystems erkannt werden. Weitere im MHC kodierte Genprodukte sind an der Bildung von Peptidfragmenten und deren Transport beteiligt.

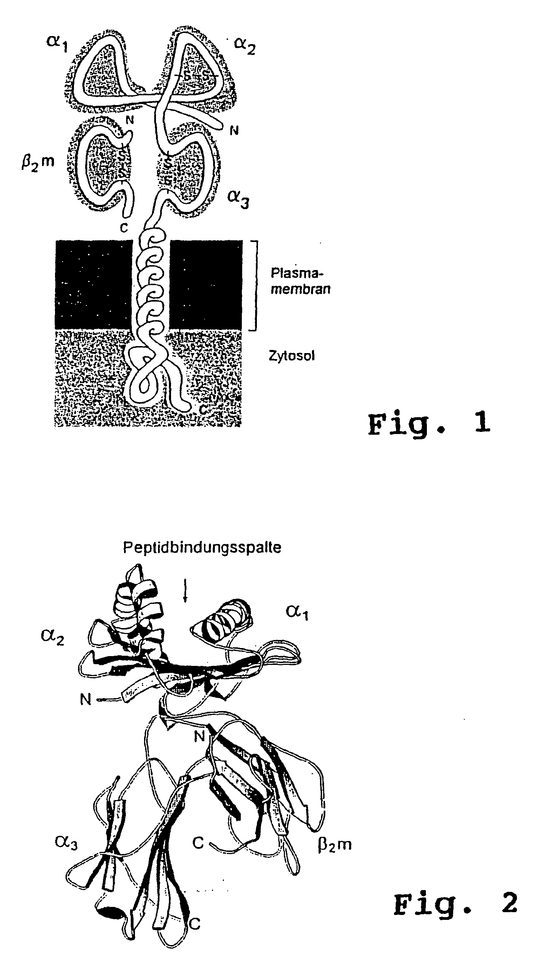

HLA Klasse I Moleküle bestehen aus einer membranverankerten schweren Kette (43 kDa) und einer nichtkovalent assoziierten leichten Kette, dem ß₂-Mikroglobulin (ß₂m; 12 kDa; siehe Fig. 1). Die schwere Kette setzt sich aus drei extrazellulären Domänen (α₁, α₂, α₃) mit je etwa 90 Aminosäuren, einem etwa 25 Aminosäure langen transmembranalen Bereich und einer c-terminalen, cytoplasmatischen Region zusammen. Das konservierte Asparagin an Position 86 der α₁-Domäne ist bei allen HLA Klasse I Proteinen N-glykosyliert. ß₂-Mikroglobulin ist nicht membrangebunden und besitzt nur eine Domäne.

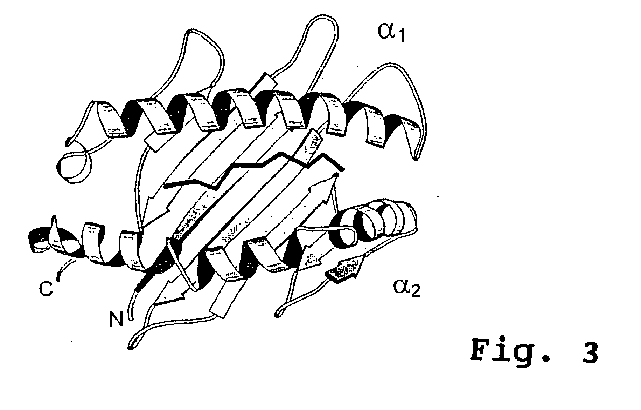

Aus röntgenkristallografischen Untersuchungen (siehe z.B. J.Mol.Biol. Vol. 285, 645-653, 1999) ist die in Fig. 2 dargestellte dreidimensionale Struktur der HLA Klasse I Moleküle bekannt. ß₂-Mikroglobulin und die membran-proximale Domäne der schweren Kette (α₃), die die für Moleküle der Immunglobulin-Superfamilie charakteristische Faltung besitzen, stützen und stabilisieren die von der α₁- und α₂-Domäne gemeinsam gebildete Peptidbindungsspalte, in der das Peptid zwischen zwei α-Helices, die auf einer von 8 antiparallelen ß-Strängen gebildeten Fläche liegen, eingeschlossen wird (Fig. 3).

Die schwere HLA Kette unterliegt einem hohen Polymorphismus, ß₂m hingegen ist in allen HLA Klasse I Molekülen identisch und wird außerhalb des HLA Komplexes auf dem Chromosom 15 kodiert. Bisher sind etwa 70 HLA-A Allele, etwa 200 HLA-B Allele und etwa 70 HLA-C Allele bekannt, von denen jedes Individuum maximal zwei Allele pro Genlocus exprimieren kann. Die Variabilität der Allele ist fast vollständig auf die α₁- und α₂-Domänen beschränkt und betrifft hauptsächlich Aminosäuren, deren Seitenketten in die Peptidbindungsspalte oder zum T-Zell Rezeptor zeigen.

Der Boden der Peptidbindungsspalte besteht aus 6 unterschiedlich stark ausgeprägten Vertiefungen oder Taschen, die von den Aminosäure-Seitenketten des HLA-Proteins gebildet werden. Die präsentierten Peptide weisen in der Regel eine Länge von 8 oder 9 Aminosäuren auf und werden in einer gestreckten Konformation gebunden (siehe Fig. 3). Ein konserviertes Netzwerk an Wasserstoffbrücken positioniert die freien N- und C-Termini in den Taschen an den Enden der Bindungsspalte und bestimmt somit die Orientierung der Peptide. Zusätzliche allelspezifische Kontakte bestehen zwischen den Seitenketten des Peptids und den polymorphen Aminosäuren des HLA Proteins. Die von letzterem gebildeten Taschen können einzelne Aminosäurereste des Peptides, die nach unten in die Bindungsspalte zeigen, vollständig aufnehmen. Diese Seitenketten werden auch als "anchor residues" bezeichnet, da sie das Peptid fest in der Bindungsspalte verankern. Jedes HLA Protein besitzt mindestens eine von polymorphen Aminosäuren geformte, tiefe Tasche, die spezifisch für das jeweilige Allel ist. Nur Peptide mit passenden "anchor residues" können gebunden werden.

Beide Ketten eines HLA Klasse I Moleküls werden in der Zelle getrennt synthetisiert und cotranslational in das Endoplasmatische Retikulum (ER) befördert. Hier erfolgt die Assemblierung der schweren Kette (HC, Heavy Chain) mit ß₂-Mikroglobulin (ß₂m) und einem Peptid zu funktionellen Komplexen. An diesem koordinierten Faltungsprozeß sind eine Reihe unterschiedlicher Proteine beteiligt. (Immunol.Today 21, (2000) 83-88.

Dieser intrazelluläre Vorgang kann in vitro in ähnlicher Weise nachvollzogen werden, indem die drei Komponenten des trimolekularen Endproduktes, d.h. eines HLA Klasse I Moleküls, in einem geeigneten Medium miteinander zur Interaktion gebracht werden. Es ist in diesem Zusammenhang möglich, die für diese drei Komponenten kodierenden DNA-Moleküle in einem, zwei oder drei Konstrukten zur Expression zu bringen, so daß die drei Komponenten entweder einzeln oder als dimeres bzw. als trimeres Protein zur weiteren Verarbeitung vorliegen können. Unter den bekannten Allelen wird eine Auswahl dergestalt getroffen, daß einem möglichst seltenen Allel (d.h. bei weniger als 5 % aller Personen der Weltbevölkerung vorkommend) der Vorzug vor einem in der Bevölkerung häufiger vorkommenden Allel gegeben wird. Die Allele HLA-A*6601 bzw. HLA-B*7301 können als Beispiel dienen, da sie extrem selten vorkommen (bei unter 1 % der Mitteleuropäer). Es ist hierbei zu beachten, daß Sequenzunterschiede zwischen HLA Klasse I Allelen in aller Regel mehr als nur eine einzige Nukleotidposition betreffen, so daß die entsprechenden Proteinprodukte sich in einer variablen Anzahl von Aminosäuren unterscheiden können. Diese Unterschiede führen charakteristischerweise zu unterschiedlichem Peptidbindungsverhalten. Als Beispiel sollen die beiden HLA Moleküle HLA-B35 und HLA-B53 dienen, die sich nur durch das Vorhandensein der Bw6- (B35) bzw. Bw4- (B53) Determinante unterscheiden, d.h. durch fünf geänderte Aminosäuren. Die gebundenen Peptide enthalten in der Regel als C-terminalen "Anchor" ein Tyrosin (B35), während mehrere Aminosäuren als C-Terminus des Peptids von HLA-B53 Molekülen toleriert werden. Der andere "Anchor" in der Peptidposition 2 ist hingegen bei beiden Molekülen ein Prolin, bedingt durch die Identität der Moleküle im Bereich der B-Tasche, welche dieses Prolin bindet.

Die für die Auswahl von Allelen und Peptiden relevanten Daten sind dem Fachmann und der Allgemeinheit zugänglich (u.a. im Internet unter http://www.ebi.ac.uk/imgt/hla/ für DA- und Proteinsequenzen von HLA Molekülen; ftp: //ftp.wehi.deu.au/pub/biology/mhcpep/ oder http//134.2.96.221/scripts/hlaserver.dll/home.htm für Peptidsequenzen). Daraus kann der Fachmann auch entnehmen, welche Allele welche Merkmale tragen und mit welcher Häufigkeit sie auftreten.

Eine weitere Ausführungsform der Erfindung besteht darin, artifizielle HLA Klasse I Allele zu konstruieren und die dazugehörigen Proteine zu synthetisieren, welche in der menschlichen Bevölkerung nach heutiger Kenntnis nicht vorkommen und die gegebenenfalls ein geändertes Peptidbindungsverhalten haben. So ist es möglich, durch in vitro-Mutagenese die B-Tasche des HLA-B27 Moleküls, die preferentiell ein Arginin der Peptidposition 2 bindet, durch die B-Tasche des HLA-A2 Moleküls zu ersetzen, was zur Bindung von Peptiden führt, die eine hydrophobe Aminosäure, z.B. Leucin, in der Peptidposition 2 enthalten. Der Begriff "Allele" erfaßt daher sowohl natürliche als auch artifizielle Allele.

Nach der Auswahl erfolgt erfindungsgemäß die Synthese des löslichen, extrazellulären Anteils des Proteins (der Proteine) d.h. der α₁, α₂ und α₃ Domänen, und dann die in vitro-Assemblierung in der Weise, daß bei Vorhandensein von ß₂m in einer Rekonstitutionslösung bei einer Temperatur im Bereich von 0 bis 40 °C das Protein des ausgewählten HLA Allels in einen Peptidpool mit unterschiedlichen Peptiden aus 7 bis 12 Aminosäuren, vorzugsweise 8 bis 9 Aminosäuren, eingebracht und für einen Zeitraum von 1 bis 7 Tagen inkubiert wird. Der Peptidpool enthält insbesondere Peptide mit geeigneten "Anchors" (Ankeraminosäuren, s.o.), etwa Prolin an Position 2 und Tyrosin an Position 8 oder 9 eines Peptids im Falle des HLA-B35 Moleküls. Als ein solches Peptid kann beispielsweise eingesetzt werden das Oktamer VPLRPMTY oder das Nonamer LPPLDITPY (J.Mol.Biol. 285, 645-653, 1999).

Das rekonstituierte, trimere HLA Klasse I Molekül wird anschließend chromatografisch, z.B. durch Hochleistungs-Flüssigchromatografie (HPLC) oder durch FPLC von den weiteren Bestandteilen des Gemisches, wie ungebundene Peptide, ß₂m usw. abgetrennt. Die korrekte Faltung des rekonstituierten Moleküls und damit native Konformation wird z.B mit Hilfe von konformationsabhängigen Antikörpern im ELISA-Test und mittels Immunpräzipitation nachgewiesen (Eur.J.Immunol. Vol.23, 734-738, 1993; Hum. Immunol. 61, 408-418, 2000).

In einer besonderen Ausführungsform der Erfindung wird das nach Punkt c) oder d) gebildete HLA Klasse I Molekül vor der nachfolgenden Fragmentierung für einen Zeitraum von 1-36 Stunden, vorzugsweise 18-36 Stunden bei 4 bis 40 °C, vorzugsweise 37 °C in ein Warmblüterserum gegeben, vorzugsweise in ein Mäuseserum, insbesondere in ein Serum von ß₂m(-/-) Mäusen. Dadurch werden gegebenenfalls weitere Substanzen an das HLA Molekül gebunden.

Bei der anschließenden Fragmentierung des gereinigten HLA Klasse I Moleküls kann eine Vielzahl von Proteasen verwendet werden.

Bevorzugt werden die Proteasen ausgewählt unter Proteinasen wie Serin-Proteinasen, Cystein-Proteinasen, Aspartat-Proteinasen und Metall-Proteinasen sowie auch Peptidasen, wie Aminopeptidasen, Dipeptidasen, Dipeptidylcarboxypeptidasen, Carboxypeptidasen, Omega-Peptidasen. Bevorzugt ist z.B. Pronase von Streptomyces griseus (Sigma Pronase, Typ XIV), Proc.Natl.Acad.Sci.USA Vol.96, 1522-1525, 1999.

Die Fragmentierung mit Pronase kann beispielsweise bei Raumtemperatur für 2 Stunden erfolgen, wobei die Konzentration in Abhängigkeit von den experimentellen Gegebenheiten variieren kann.

Zur Rekonstitution löslicher HLA-A oder HLA-B/Peptid Komplexe kann gegebenenfalls ein Rekonstitutionspuffer hinzugegeben werden, beispielsweise 400 mM L-Arg-HCl, 2 mM EDTA, 5 mM reduziertes Gluthation, 0,55 mM oxidiertes Gluthation, 100mM Tris/HCl, pH 7,5.

Nach Abtrennung der geruchlich aktiven Stoffe, beispielsweise durch chromatographische Verfahren (HPLC, FPLC) oder immunologische Verfahren, können diese Stoffe in entsprechenden Konzentrationen in kosmetische Zubereitungen wie Parfüms, Emulsionen, Seifen, öle, Gele, Cremes und dergleichen eingebracht werden. Derartige Formulierungen bilden ebenfalls einen Gegenstand der Erfindung.

Eine Besonderheit der erfindungsgemäßen Duftkomposition besteht darin, daß sie aus zwei getrennt formulierten Produkten bestehen kann. Es können auch drei getrennte Produkte formuliert werden.

In der bevorzugten erfindungsgemäßen Duftkomposition bildet das nach Anspruch 1 hergestellte Produkt eine kombinatorische Einheit mit einem zweiten Produkt, das ebenfalls nach dem Verfahren gemäß Anspruch 1 hergestellt ist mit der Maßgabe, daß das ausgewählte Allel für das zweite Produkt ein anderes Allel ist als das Allel für das erste Produkt. Dabei unterscheidet sich das Allel für das zweite Produkt ebenfalls durch wenigstens ein Merkmal von anderen Allelen der HLA Klasse I Moleküle. Dieses Merkmal ist ein sog. seltenes Merkmal, das bei weniger als 5 %, vorzugsweise weniger als 1% der Weltbevölkerung auftritt (Allelfrequenz).

Die kombinatorische Einheit umfaßt zwei Duftformulierungen, von denen jeweils eine für unterschiedliche Partner, vorzugsweise unterschiedliche Partner verschiedenen Geschlechtes vorgesehen oder zuzuordnen ist.

Bevorzugte Duftkompositionen sind dadurch gekennzeichnet, daß die Allele HLA-A*6601 und HLA-B*7301; die Allele HLA-B*1301 und HLA-B*2709; die Allele HLA-B*2705 mit Ersatz der "B"-Tasche durch die "B"-Tasche des HLA-A*0201 Allels und HLA-B*7301; als Ausgangsstoffe für getrennte Duftkompositionen eingesetzt werden. Dabei bilden die genannten Allelpaare Ausgangsstoffe für entsprechende Duftkompositionspaare z.B. A und B, von denen der Duft A dem Partner A und der Duft B dem Partner B zugeordnet wird, um die Attraktivität der Partner A und B füreinander zu erhöhen.

Die Erfindung wird nachstehend durch Beispiele näher erläutert. In den dazugehörigen Zeichnungen zeigen
Fig. 1 eine schematische Darstellung eines HLA Klasse I Molekül mit extrazellulären Domänen α₁, α₂, α₃; die membranverankerte schwere Kette ist nicht kovalent mit ß₂m assoziiert.
Fig. 2 eine Struktur des extrazellulären Anteils eines HLA Klasse I Moleküls.
Fig. 3 einen Blick von oben auf die Peptidbindungstasche mit dem zickzackförmig eingebundenen Peptid (HLA-B*3501, mit Peptid LPPLDITPY).

### Beispiel 1 Herstellung der Duftkomponente A

### Induktion

LB-Medium = 10 g Bacto-Trypton, 5 g Yeast Extract, 10 g NaCl gelöst in 1 1 ddH₂O.
300 ml LB/Amp-Medium wurden in einem 1 1 Kolben mit 3 ml Übernacht-Kultur (bei Raumtemperatur) angeimpft und bei 37 °C bis zu einer OD₅₆₀ von 0,7 bei 300 U/Min. geschüttelt. Nach Einstellen der IPTG (IPTG = Isopropyl-ß-D-thiogalactopyranosid) Konzentration auf 0,4 mM wuchsen die Bakterien weitere 4 Stunden bei 37 °C, bevor sie bei 2700 g für 20 Minuten zentrifugiert wurden. Das Pellet wurde zur Präparation von "Inclusion Bodies" eingesetzt.

Zur Identifizierung proteinexprimierender Klone nach einer Transformation wurde eine Induktion in kleinerem Maßstab durchgeführt. Hierbei wurden je 200 µl LB/Amp in einer 96 Loch Zell-Kulturplatte (Costar) mit einer Kolonie angeimpft und über Nacht bei Raumtemperatur inkubiert. Danach wurden 100 µl abgenommen und mit 100 µl LB/Amp/2xIPTG in eine neue Vertiefung gefüllt und 4 Stunden bei 37 °C stehengelassen. Anschließend wurde die Kultur in einem Eppendorfgefäß zentrifugiert, die Bakterien in 100 µl SDS-Probenpuffer aufgenommen (SDS = Natriumlaurylsulfat), 10 Minuten bei 95 °C lysiert und ein Aliquot auf ein SDS-Polyacrylamidgel aufgetragen. Proteinexprimierende Klone zeigten eine zusätzlich Bande.

### Präparation von "Inclusion Bodies"

Fremde Proteine werden in bakterien häufig als denaturiertes Präzipitat im Zytoplasma abgelagert. Diese "Inclusion Bodies" lassen sich nach dem Zellaufschluß durch mehrere Zentrifugationsschritte reinigen, in Harnstoff lösen und dann zu funktionellen Proteinen rekonstituieren.

Das nach der Bakterieninduktion gewonnene Pellet wurde in 10 ml Lysepuffer (25 % Sucrose/ 1mM EDTA/ 50 mM Tris/HCl, pH 8,0) und 1mM Phenylmethansulfonsäurefluorid aufgenommen und über Nacht bei -20 °C eingefroren oder sofort weiterverarbeitet. Nach Zugabe von 0,5 ml Lysozym (10 mg/ml Lyspuffer) und Inkubation für 30 Minuten auf Eis nahm die Lösung auf Grund der DNS-Freisetzung eine viskose Konsistenz an. Der Zusatz von MgCl₂ (auf 10 mM), MnCl₂ (auf 1 mM) und DNase I (auf 10 µg/ml) verflüssigte das Lysat wieder. Nach einer Zentrifugation für 10 Min bei 10.000 g wurde das Pellet in 10 ml Detergenspuffer (0,2 M NaCl/1 % Desoxycholsäure (Sigma)/ 1 % Nonidet P-40 (Sigma)/ 2 mM EDTA/ 2 mM DTT (Dithiothreitol)/ 20 mM Tris/HCl, pH 7,5) mittels Ultraschall resuspendiert und nach einer Inkubation für 10 Minuten auf Eis erneut zentrifugiert. Die Inclusion Bodies wurden 4x in Tritonpuffer (100 mM NaCl/ 1mM EDTA/ 2mM DTT/ 0,5% TritonX100 (Serva)/ 50 mM Tris/HCl, pH 8,0) gewaschen und in 4 ml 20 mM Tris/HCl, pH 7,5/.150 mM NaCl/ 10 mM DTT aufgenommen.

### Rekonstitution des funktionellen HLA/Peptid-Komplexe

Das funktionelle Molekül HLA A*6601 wurde aus der in Harnstoff gelösten schweren Kette (α₁, α₂, α₃ Domäne), ß₂-Mikroglobulin und dem Peptid 
 T       R
X XXXXXX
 V       K
 nach dem Verdünnungsprotokoll von V K Garboczi et al. (Proc.Natl.Acad.Sci.USA Vol. 89, 3429-3433, 1992) rekonstituiert.

Die in Form von "Inclusion Bodies" gereinigten Untereinheiten wurden in frisch angesetztem und filtriertem Harnstoffpuffer (50 % Harnstoff/ 50 mM NaCl/ 20 mM Tris/HCl, pH 7,5) gelöst (30 Min bei Raumtemperatur im Schüttler), zentrifugiert (20 Min, 10.000 g) und ihre Konzentration bestimmt. Beide Ketten wurden vereinigt, mit Harnstoffpuffer auf 5 ml aufgefüllt und in 200 ml Rekonstitutionspuffer (400 mM 1-Arginin/HCl (Sigma)/ 2mM EDTA/ 5 mM reduziertes Gluthathion/ 0,5 mM oxidiertes Gluthation, 100mM Tris/HCl, pH 7,5), der das Peptid enthielt, verdünnt. Die Endkonzentration der HLA Kette betrug 1 µM, die des ß₂-Mikroglobulins 2 µM und des Peptides 10 µM. Der Ansatz verblieb für 36 Stunden bei 4 °c und wurde nach Konzentrierung auf 1 ml mit Hilfe von Centriprep-10 Konzentrierröhrchen (Amicon) auf einer Gelfiltrationssäule aufgetrennt.

### Gelfiltration

Die Reinigung des HLA/Peptid-Komplexes erfolgte mittels einer mit 20 mM Tris/HCl, pH 7,5/ 150 mM NaCl äquilibrierten Superdex-75-HR Säule (Pharmacia), auf der der konzentrierte Restitutionsansatz bei einer Flußrate von 1 ml/min aufgetrennt wurde.

### Fragmentierung

1mg des gereinigten HLA/Peptid-Komplexes in 1ml Puffer (20 mM Tris/HCl , pH 7,5/ 150 mM NaCl) wird entweder direkt mit bakterieller Pronase Typ XIV aus Streptomyces griseus versetzt (Endkonzentration 0,1 mg/ml) oder in Serum von ß₂m (-/-) Mäusen, die auf Grund defekter Gene für ß₂m kein ß₂m-Protein mehr produzieren können, gegeben (Endkonzentration des HLA/Peptid-Komplexes lmg/ml). Vor Zugabe des Enzyms kann eine Inkubation der HLA/Peptid-Komplexe im Serum bei 4-40 °C für bis zu 24 Stunden erfolgen; dann wird mit bakterieller Pronase Typ XIV aus Streptomyces griseus versetzt (Endkonzentration 2 mg/ml). Die Degradation der Proteine wird bei Raumtemperatur für 2 Stunden durchgeführt. Andere Konzentrationen des HLA/Peptid-Komplexes und des Enzyms sowie der Degradationszeit und -temperatur sind möglich. Die fragmentierten Proteine werden entweder bei -80 °C eingefroren oder direkt weiterverarbeitet.

### Abtrennung

Die Abtrennung der geruchlich inaktiven Bestandteile kann mittels Sephadex G-100 im Batch-Verfahren erfolgen. Hierbei wird die Probe, welche die degradierten Proteine enthält, mit Sephadex (1/10 des Volumens) gemischt, 10 min bei Raumtemperatur inkubiert und kurz zentrifugiert. Der überstand enthält die geruchlich aktiven Bestandteile der Probe.
Auf diese Weise erhielt man die Duftkomponente A.

### Beispiel 2 Herstellung der Duftkomponente B

Es wurde wie im Beispiel 1 gearbeitet, jedoch als Allel wurde HLA*7301 und als Peptid XRXXXXXXP ausgewählt. Die nachfolgenden Schritte entsprachen denen des Beispiels 1 und man erhielt nach der Fragmentierung und Abtrennung die Duftkomponente B.

### Beispiel 3 Parfümkomposition (Eau de Parfume)

### Komposition A

Bei Raumtemperatur wurden die folgenden Bestandteile miteinander vermischt (Gewichtsprozent).

| | |
|---|---|
| Duftkomponente A | 11 % |
| Ethanol | q.s. ad 100 |
| Wasser | 1 % |
| Color blue | 0,05 % |

Gleichzeitig wurden die folgenden Bestandteile miteinander vermischt.

### Komposition B

| | |
|---|---|
| Duftkomponente B | 10 % |
| Ethanol | q.s. ad 100 |
| Wasser | 1 % |
| Color yellow | 0,06 % |

Beide Kompositionen wurden danach 10 Tage bei 5 bis 10°C gelagert und als eine Verkaufseinheit konfektioniert. Anschließend wurde Komposition A einem Partner einer Lebensgemeinschaft und Komposition B dem anderen Partner der Lebensgemeinschaft zur Verfügung gestellt. Beide Partner beurteilten den Duft des jeweils anderen Partners als sehr erotisch und anziehend.

### Beispiel 4 Gesichts- und Körpercreme

### TEIL A

| **Phase A** (In Gewichtsprozent) | |
|---|---|
| Propylene Glycol Dicaprylate | 4 |
| Cetearyl Isononanoate | 2,5 |
| Shea Butter | 1,0 |
| Dimethicone | 1,2 |

| **Phase B** | |
|---|---|
| Wasser | q.s. ad 100 |
| Glycerine | 3 |

| **Phase C** | |
|---|---|
| Konservierungsmittel | 0,5 |
| Duftkomponente A | 1,5 |
| Farbstoff orange | 0,08 |

Die Phase A wurde auf 70 °C erwärmt und Phase B ebenfalls separat auf 70 ° C erwärmt. Danach wurden beide Phasen miteinander vermischt und das Gemisch auf 40 °C abgekühlt. Dann erfolgte die Zugabe der Phase C , und das Gesamtgemisch wurde homogenisiert.

### TEIL B

Die gleiche Zusammensetzung der Phasen A und B wie in TEIL A von Beispiel 4 wurde genommen.

| **Phase C** | |
|---|---|
| Konservierungsmittel | 0,5 |
| Duftkomponente B | 1,5 |
| Farbstoff blau | 0,08 |

Die Verarbeitung erfolgte wie für den TEIL A von Beispiel 4. Die TEILE A und B der Creme von Beispiel 4 wurden zusammen als eine Verkaufseinheit konfektioniert. Anschließend wurde TEIL A einem Partner einer Lebensgemeinschaft und TEIL B dem anderen Partner der Lebensgemeinschaft zur verfügung gestellt. Beide Partner beurteilten den Duft des jeweils anderen Partners als sehr attraktiv und anziehend.

## Patentansprüche

1. Duftkomposition aus HLA-Molekülen, hergestellt nach einem Verfahren, bei dem man
a) HLA Allele der Klasse I auswählt, die durch in-vitro Mutagenese der B-Tasche bereits vorhandener HLA Allele der Klasse I produziert wurden und natürlicherweise nicht vorkommen und somit eine Häufigkeit von 0 % haben; und
b) den löslichen, extrazellulären Anteil des Proteins, für welches das ausgewählte Allel kodiert, synthetisiert und dann einer in-vitro Assemblierung in Anwesenheit von ß₂-Mikroglobulin (ß₂m) unterzieht, indem eine Vielzahl unterschiedlicher Peptide mit einer Länge von typischerweise 7 bis 12 Aminosäuren und mit freien N- und C-Termini zur Ausbildung von löslichen HLA Klasse I Molekülen, bestehend aus den extrazellulären Domänen α₁, α₂ und α₃, ß₂m und einem Peptid, entsteht, wobei das Peptid in einer von den extrazellulären Domänen α₁ und α₂ ausgebildeten Peptidbindungstasche des HLA Moleküls gebunden vorliegt; und
c) die gebildeten HLA Klasse 1 Moleküle mit den gebundenen Peptiden in einer Reinigungsstufe von den anderen Bestandteilen abtrennt; und
d) die gereinigten HLA Klasse I Moleküle einer Fragmentierung mit einer oder mehreren Proteasen unterwirft; und
e) die bei der Fragmentierung entstandenen geruchlich aktiven Stoffe als Einzelkomponente oder als Mischung in eine kosmetische Zubereitung einbringt.

2. Duftkomposition nach Anspruch 1, **dadurch gekennzeichnet, daß** man in Punkt e) die geruchlich aktiven Stoffe nach vorheriger Abtrennung von den anderen Stoffen in die kosmetische Zusammensetzung einbringt.

3. Duftkomposition nach Anspruch 1, **dadurch gekennzeichnet, daß** die Proteasen ausgewählt sind unter Proteinasen wie Serin-Proteinasen, Cystein-Proteinasen, Aspartat-Proteinasen und Metall-Proteinasen, Aminopeptidasen, Dipeptidasen, Dipeptidylcarboxypeptidasen, Carboxypeptidasen, Omega-Peptidasen.

4. Duftkomposition nach Anspruch 1, **dadurch gekennzeichnet, daß** das nach Punkt c) oder d) gebildete HLA Klasse I Molekül vor der nachfolgenden Fragmentierung für einen Zeitraum von 1-36 Stunden bei 4 bis 40 °C in ein Serum von ß₂m(-/-) Mäusen gegeben wird.

5. Duftkomposition nach Anspruch 1, **dadurch gekennzeichnet, daß** das nach Anspruch 1 hergestellte Produkt eine kombinatorische Einheit mit einem zweiten Produkt bildet, das nach dem Verfahren gemäß Anspruch 1 hergestellt ist mit der Maßgabe, daß das ausgewählte Allel für das zweite Produkt ein anderes Allel ist als das Allel für das erste Produkt, wobei das Allel für das zweite Produkt sich ebenfalls durch wenigstens ein Merkmal von anderen Allelen der HLA Klasse I Moleküle unterscheidet, das bei weniger als 5 % der Personen der Weltbevölkerung auftritt.

6. Duftkomposition nach Anspruch 5, **dadurch gekennzeichnet, daß** die kombinatorische Einheit zwei getrennte Duftformulierungen umfaßt.

7. Duftkomposition nach Anspruch 5, **dadurch gekennzeichnet, daß** sie in einer klaren Lösung, einer Seife, einem kosmetischen Gel, einer kosmetischen Emulsion, einer Creme vorliegt, in einer Konzentration von 0,0001 bis 21 Gew-%, bezogen auf die Gesamtzusammensetzung.

8. Verfahren zur Herstellung einer Duftkomposition aus HLA-Molekülen, bei dem man
a) unter bekannten HLA Allelen der Klasse I ein Allel auswählt, das sich durch wenigstens ein Merkmal von anderen Allelen der HLA Klasse 1 Moleküle unterscheidet, das bei weniger als 5 % der Personen der Weltbevölkerung auftritt; oder
b) HLA Allele der Klasse I auswählt, die durch in-vitro Mutagenese der B-Tasche bereits vorhandener HLA Allele der Klasse I produziert wurden und natürlicherweise nicht vorkommen und somit eine Häufigkeit von 0 % haben; und
c) den löslichen, extrazellulären Anteil des Proteins, für welches das ausgewählte Allel kodiert, synthetisiert und dann einer in-vitro Assemblierung in Anwesenheit von ß₂-Mikroglobulin (ß₂m) unterzieht, indem eine Vielzahl unterschiedlicher Peptide mit einer Länge von typischerweise 7 bis 12 Aminosäuren und mit freien N- und C-Termini zur Ausbildung von löslichen HLA Klasse I Molekülen, bestehend aus den extrazellulären Domänen α₁, α₂ und α₃, ß₂m und einem Peptid, entsteht, wobei das Peptid in einer von den extrazellulären Domänen α₁ und α₂ ausgebildeten Peptidbindungstasche des HLA Moleküls gebunden vorliegt; und
d) die gebildeten HLA Klasse 1 Moleküle mit den gebundenen Peptiden in einer Reinigungsstufe von den anderen Bestandteilen abtrennt; und
e) die gereinigten HLA Klasse I Moleküle einer Fragmentierung mit einer oder mehreren Proteasen unterwirft; und
f) die bei der Fragmentierung entstandenen geruchlich aktiven Stoffe als Einzelkomponente oder als Mischung in eine kosmetische Zubereitung einbringt.

9. Verfahren nach Anspruch 8, **dadurch gekennzeichnet, daß** man in Punkt e) die geruchlich aktiven Stoffe nach vorheriger Abtrennung von den anderen Stoffen in die kosmetische Zusammensetzung einbringt.

10. Verfahren nach Anspruch 8, **dadurch gekennzeichnet, dass** die Proteasen ausgewählt sind unter Proteinasen wie Serin-Proteinasen, Cystein-Proteinasen, Aspartat-Proteinasen und Metall-Proteinasen, Aminopeptidasen, Dipeptidasen, Dipeptidylcarboxypeptidasen, Carboxypeptidasen, Omega-Peptidasen.

11. Verfahren nach Anspruch 8, **dadurch gekennzeichnet, daß** die Allele
HLA-A^{*}6601 und HLA-B^{*}7301; die Allele HLA-B^{*}1301 und HLA-B^{*}2709; die Allele HLA-B^{*}2705 mit Ersatz der "B"-Tasche durch die "B"-Tasche des HLA-A^{*}0201 Allels und HLA-B^{*}7301;
als Ausgangsstoffe für getrennte Duftkompositionspaare eingesetzt werden.

12. Verfahren nach Anspruch 8, **dadurch gekennzeichnet, daß** das nach Punkt c) oder d) gebildete HLA Klasse I Molekül vor der nachfolgenden Fragmentierung für einen Zeitraum von 1-36 Stunden bei 4 bis 40 °C in ein Serum von ß₂m(-/-) Mäusen gegeben wird.

13. Verfahren nach Anspruch 8, **dadurch gekennzeichnet, daß** mit dem nach Anspruch 8 hergestellten Produkt eine kombinatorische Einheit mit einem zweiten Produkt gebildet wird, das nach dem Verfahren gemäß Anspruch 8 hergestellt ist mit der Maßgabe, daß das ausgewählte Allel für das zweite Produkt ein anderes Allel ist als das Allel für das erste Produkt, wobei das Allel für das zweite Produkt sich ebenfalls durch wenigstens ein Merkmal von anderen Allelen der HLA Klasse I Moleküle unterscheidet, das bei weniger als 5 % der Personen der Weltbevölkerung auftritt.

14. Verfahren nach Anspruch 13, **dadurch gekennzeichnet, daß** die kombinatorische Einheit zwei getrennte Duftformulierungen umfaßt.

15. Verfahren nach Anspruch 13, **dadurch gekennzeichnet, daß** die Duftkomposition in eine klare Lösung, eine Seife, ein kosmetisches Gel, eine kosmetische Emutsion, eine Creme eingebracht wird, in einer Konzentration von 0,0001 bis 21 Gew-%, bezogen auf die Gesamtzusammensetzung.

16. Duftkomposition, hergestellt nach dem Verfahren gemäß Anspruch 13, wobei
sie in einer klaren Lösung, einer Seife, einem kosmetischen Gel, einer kosmetischen Emulsion, einer Creme vorliegt, in einer Konzentration von 0,0001 bis 21 Gew-%, bezogen auf die Gesamtzusammensetzung.

## Claims

1. A perfume composition of HLA molecules prepared according to a method whereby
a) HLA alleles of class I are selected which are produced by in-vitro mutagenesis of the B pocket of existing HLA alleles of class I and which do not occur naturally and thus have an incidence of 0 %; and
b) the soluble extra-cellular part of the protein for which the selected allele codes is synthesised and then subjected in-vitro to assembly in the presence of β₂-microglobulin (β₂m) by using a plurality of different peptides having a length of typically 7 to 12 amino acids and with free N- and C-termini to form soluble HLA class I molecules consisting of the extracellular domains α₁, α₂ and α₃, β₂m and a peptide is formed, whereby the peptide is present in bound form in a peptide binding chain of the HLA molecule formed by the extracellular domains α₁ and α₂; and
c) the HLA class I molecules thus formed together with the bound peptides are separated from the other constituents in one purification step; and
d) the purified HLA class I molecules are subjected to fragmentation with one or more proteases; and
e) the substances that are active in producing the odor and are formed in fragmentation are added either as an individual component or as a mixture to a cosmetic preparation.

2. The perfume composition according to claim 1, wherein the substances that are active in producing the odor after previous separation from the other substances are included in the cosmetic preparation.

3. The perfume composition according to claim 1, wherein the proteases are selected from proteases such as serine proteases, cysteine proteases, aspartate proteases and metal proteases, amino peptidases, dipeptidases, dipeptidyl carboxypeptidases, carboxypeptidases, omega-peptidases.

4. The perfume composition according to claim 1, wherein the HLA class I molecule formed according to point c) or d) is added to a serum from β₂m (-/-) mice for a period of 1 to 36 hours at 4 C to 40°C before subsequent fragmentation.

5. The perfume composition according to claim 1, wherein the product produced according to claim 1 forms a combination unit with a second product which is produced by the method according to claim 1 with the provision that the selected allele for the second product is a different allele from the allele for the first product, the allele for the second product also differs by at least one feature by other alleles of the HLA class I molecules, and it occurs in less than 5 % of the world's population.

6. The perfume composition according to claim 5, wherein the combination unit includes two separate perfume formulations.

7. The perfume composition according to claim 5, wherein it is in the form of a clear solution, soap, cosmetic gel, cosmetic emulsion, cream, and in a concentration of 0.0001 to 21 wt %, based on the total composition.

8. Process for manufacturing a perfume composition of HLA molecules in which
a) an allele is selected from known HLA alleles of class I such that it differs by at least one feature from other alleles of the HLA class I molecules which occurs in less than 5 % of the world's population; or
b) HLA alleles of class I are selected which have been produced by in-vitro mutagenesis of the B pocket of existing HLA alleles of class I and do not occur naturally and thus have an incidence of 0 %; and
c) the soluble extracellular part of the protein for which the selected allele codes is synthesised and then is subjected to an in-vitro assembling operation in the presence of β₂-microglobulin (β₂m) by using a plurality of different peptides having a length of typically 7 to 12 amino acids and with free N- and C-termini to form soluble HLA class I molecules consisting of the extra-cellular domains α₁, α₂ and α₃, β₂m and a peptide is formed, whereby the peptide is present in bound form in a peptide binding chain of the HLA molecule formed by the extracellular domains α₁ and α₂; and
d) the HLA class I molecules thus formed together with the bound peptides are separated from the other constituents in one purification step; and
e) the purified HLA class I molecules are subjected to fragmentation with one or more proteases; and
f) the substances that are active in producing the odor and are formed in fragmentation are added either as an individual component or as a mixture to a cosmetic preparation.

9. Process according to claim 8, wherein the substances that are active in producing the odor after previous separation from the other substances are included in the cosmetic preparation.

10. Process according to claim 8, wherein the proteases are selected from proteases such as serine proteases, cysteine proteases, aspartate proteases and metal proteases, amino peptidases, dipeptidases, dipeptidyl carboxypeptidases, carboxypeptidases, omega-peptidases.

11. Process according to claim 8, wherein the alleles HLA-A*6601 and HLA-B*7301, the alleles HLA-B*1301 and HLA-B*2709, the alleles HLA-B*2705 with the replacement of the "B" pocket by the "B" pocket of the HLA-A*0201 allele and HLA-B*7301, are used as starting materials for separate perfume composition pairs.

12. Process according to claim 8, wherein the HLA class I molecule formed according to point c) or d) is added to a serum from β₂m (-/-) mice for a period of 1 to 36 hours at 4 °C to 40 °C before subsequent fragmentation.

13. Process according to claim 8, wherein the product produced according to claim 1 forms a combination unit with a second product which is produced by the method according to claim 1 with the provision that the selected allele for the second product is a different allele from the allele for the first product, the allele for the second product also differs by at least one feature by other alleles of the HLA class I molecules, and it occurs in less than 5 % of the world's population.

14. Process according to claim 13, wherein the combination unit includes two separate perfume formulations.

15. Process according to claim 13, wherein the perfume composition is in the form of a clear solution, soap, cosmetic gel, cosmetic emulsion, cream, and in a concentration of 0.0001 to 21 wt %, based on the total composition.

16. Perfume composition prepared according to the process of claim 13, which composition is in the form of a clear solution, soap, cosmetic gel, cosmetic emulsion, cream, and in a concentration of 0.0001 to 21 wt %, based on the total composition.

## Revendications

1. Composition odoriférante constituée de molécules de HLA, produite selon un procédé, dans le cadre duquel
a) on choisit des allèles de classe I qui ont déjà été produits par mutagenèse *in vitro* d'allèles de HLA de classe I déjà présents de la poche B et ne sont pas présents à l'état naturel et ainsi, ont une fréquence de 0 % ; et
b) on synthétise la partie extracellulaire soluble de la protéine pour laquelle l'allèle choisi code, et ensuite, on la soumet à un assemblage in vitro en présence de microglobuline β₂ (β₂m), ce qui donne une multiplicité de peptides différents présentant une longueur habituellement de 7 à 12 acides aminés et des terminaisons N et C libres pour former des molécules de HLA de classe I solubles constituées des domaines extracellulaires α₁, α₂ et α₃, β₂m et un peptide, le peptide étant lié dans l'une des poches de liaison peptidique constituées des domaines extracellulaires α₁ et α₂ de la molécule HLA ; et
c) on sépare les molécules de HLA de classe I formées avec les peptides liés dans une étape de purification des autres composants ; et
d) on soumet les molécules de HLA de classe I purifiées à une fragmentation avec une ou plusieurs protéases ; et
e) on intègre les substances actives d'un point de vue odoriférant, obtenues lors de la fragmentation, en tant que composants individuels ou en mélange dans une préparation cosmétique.

2. Composition odoriférante selon la revendication 1, **caractérisée en ce que** l'on intègre les substances actives d'un point de vue odoriférant du point e) après séparation préalable des autres substances dans la préparation cosmétique.

3. Composition odoriférante selon la revendication 1, **caractérisée en ce que** les protéases sont choisies parmi les sérine-protéinases, les cystéine-protéinases, les aspartate-protéinases et les protéinases métalliques, les aminopeptidases, les dipeptidases, les dipeptidylcarboxypeptidases, les carboxypeptidases, les oméga-peptidases.

4. Composition odoriférante selon la revendication 1, **caractérisée en ce que** la molécule de HLA de classe I formée selon le point c) ou d) est placée avant la fragmentation suivante pendant une durée de 1 à 36 heures de 4 à 40° C dans un sérum de souris β₂m (-/-).

5. Composition odoriférante selon la revendication 1, **caractérisée en ce que** le produit obtenu selon la revendication 1 forme une unité combinatoire avec un deuxième produit qui est obtenu avec le procédé selon la revendication 1, à condition que l'allèle choisi pour le deuxième produit soit un autre allèle que l'allèle du premier produit, l'allèle pour le deuxième produit se distinguant également par au moins une caractéristique d'autres allèles de molécules HLA de classe I qui est présente chez moins de 5 % des personnes de la population mondiale.

6. Composition odoriférante selon la revendication 5, **caractérisée en ce que** l'unité combinatoire comprend deux formulations odoriférantes séparées.

7. Composition odoriférante selon la revendication 5, **caractérisée en ce qu'**elle est présente dans une solution limpide, dans un savon, dans un gel cosmétique, dans une émulsion cosmétique, dans une crème, en une concentration allant de 0,0001 à 21 % en poids, par rapport à la composition totale.

8. Procédé de production d'une composition odoriférante constituée de molécules de HLA, dans le cadre duquel
a) on choisit parmi les allèles de HLA de classe I connus, un allèle qui se distingue par au moins une caractéristique d'autres allèles de molécules de HLA de classe I qui est présente chez moins de 5 % des personnes de la population mondiale ; ou
b) on choisit des allèles de HLA de classe I qui ont été produits par mutagenèse *in vitro* des allèles de HLA de classe I déjà présents de poche B et qui ne sont pas présents à l'état naturel et ainsi, ont une fréquence de 0 % ; et
c) on synthétise la partie extracellulaire soluble de la protéine pour laquelle l'allèle choisi code et ensuite, on la soumet à un assemblage *in vitro* en présence de microglobuline β₂ (β₂m), ce qui donne une multiplicité de peptides différents présentant une longueur habituellement de 7 à 12 acides aminés et des terminaisons N et C libres pour former des molécules de HLA de classe I solubles constituées des domaines extracellulaires α₁, α₂ et α₃, β₂m et d'un peptide, le peptide étant lié dans l'une des poches de liaison peptidique constituées des domaines extracellulaires α1 et α₂ de la molécule HLA ; et
d) on sépare la molécule de HLA de classe I formée avec les peptides liés dans une étape de purification des autres composants ; et
e) on soumet les molécules de HLA de classe I purifiées à une fragmentation avec une ou plusieurs protéases ; et
f) on intègre les substances actives d'un point de vue odoriférant, obtenues lors de la fragmentation, en tant que composants individuels ou en mélange dans une préparation cosmétique.

9. Procédé selon la revendication 8, **caractérisé en ce que** l'on intègre les substances actives d'un point de vue odoriférant au point e) après séparation préalable des autres substances dans la composition cosmétique.

10. Procédé selon la revendication 8, **caractérisé en ce que** les protéases sont choisies parmi les sérine-protéinases, les cystéine-protéinases, les aspartate-protéinases et les protéinases métalliques, les aminopeptidases, les dipeptidases, les dipeptidylcarboxypeptidases, les carboxypeptidases, les oméga-peptidases.

11. Procédé selon la revendication 8, **caractérisé en ce que** les allèles HLA-A'6601 et HLA-B'7301 ; les allèles HLA-B'1301 et HLA-B'2709 ; les allèles HLA-B'2705 avec substitution de la poche "B" par la proche "B" de l'allèle HLA-A'0201 et HLA-B'7301 ;
sont utilisés comme substances de départ de paires de composition odoriférantes séparées.

12. Procédé selon la revendication 8, **caractérisé en ce que** la molécule de HLA de classe I formée au point c) ou d) est placée avant la fragmentation suivante pendant une durée de 1 à 36 heures de 4 à 40° C dans un sérum de souris β₂m (-/-).

13. Procédé selon la revendication 8, **caractérisé en ce qu'**avec le produit formé selon la revendication 8, une unité combinatoire est formée avec un deuxième produit qui est obtenu par le procédé selon la revendication 8 à condition que l'allèle choisi pour le deuxième produit soit un autre allèle que l'allèle du premier produit, l'allèle pour le deuxième produit se distinguant également par au moins une caractéristique d'autres allèles de molécules HLA de classe I qui est présente chez moins de 5 % des personnes de la population mondiale.

14. Procédé selon la revendication 13, **caractérisé en ce que** l'unité combinatoire comprend deux formulations odoriférantes séparées.

15. Procédé selon la revendication 13, **caractérisé en ce que** la composition odoriférante est intégrée dans une solution limpide, dans un savon, dans un gel cosmétique, dans une émulsion cosmétique, dans une crème, en une concentration allant de 0,0001 à 21 % en poids, par rapport à la composition totale.

16. Composition odoriférante produite par le procédé selon la revendication 13,
la composition étant présente dans une solution limpide, dans un savon, dans un gel cosmétique, dans une émulsion cosmétique, dans une crème, en une concentration allant de 0,0001 à 21 % en poids, par rapport à la composition totale
